(19) Europäisches Patentamt
European Patent Office
Office européen des brevets

(11) Numéro de publication: **0 428 441 B1**

(12) **FASCICULE DE BREVET EUROPEEN**

(45) Date de publication de fascicule du brevet: **24.08.94**

(51) Int. Cl.$^5$: **A61K 7/13**, C07D 209/08, C07D 209/42

(21) Numéro de dépôt: **90403177.0**

(22) Date de dépôt: **08.11.90**

Le dossier contient des informations techniques présentées postérieurement au dépôt de la demande et ne figurant pas dans le présent fascicule.

(54) **Compositions tinctoriales pour fibres kératiniques contenant des précurseurs de colorants par oxydation et des coupleurs dérivés du 4-hydroxyindole, et procédé de teinture les mettant en oeuvre.**

(30) Priorité: **10.11.89 FR 8914794**

(43) Date de publication de la demande:
**22.05.91 Bulletin 91/21**

(45) Mention de la délivrance du brevet:
**24.08.94 Bulletin 94/34**

(84) Etats contractants désignés:
**AT BE CH DE DK ES FR GB GR IT LI NL SE**

(56) Documents cités:
**EP-A- 0 105 763**
**EP-A- 0 271 186**
**EP-A- 0 360 638**
**DE-A- 2 906 551**
**DE-A- 3 031 709**
**FR-A- 2 272 663**
**FR-A- 2 636 237**
**US-A- 4 013 404**

**S.T.N. SERVEUR DE BASES DE DONNEES, Karlsruhe, DE, FICHIER REGISTRY;& RN=19499-83-3**

(73) Titulaire: **L'OREAL**
**14, Rue Royale**
**F-75008 Paris (FR)**

(72) Inventeur: **Junino, Alex**
**16, rue Docteur Bergonié**
**F-93180 Livry-Gargan (FR)**
Inventeur: **Vandenbossche, Jean-Jacques**
**37, Avenue Berlioz**
**F-93270 Sevran (FR)**
Inventeur: **Richard, Hervé**
**48, rue de l'Ermitage**
**F-75020 Paris (FR)**
Inventeur: **Cotteret, Jean**
**15, Allée des Meuniers**
**F-78480 Verneuil-sur-Seine (FR)**

(74) Mandataire: **Casalonga, Axel et al**
**BUREAU D.A. CASALONGA - JOSSE**
**Morassistrasse 8**
**D-80469 München (DE)**

EP 0 428 441 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention est relative à de nouvelles compositions tinctoriales pour fibres kératiniques et en particulier pour cheveux humains, contenant des précurseurs de colorants d'oxydation et des coupleurs dérivés du 4-hydroxyindole et aux procédés de teinture mettant en oeuvre de telles compositions.

De nombreux brevets et demandes de brevets enseignent l'utilisation de dérivés indoliques à titre de colorants d'oxydation en association avec divers systèmes ou agents oxydants, tels que EP 0 360 638 et EP 0 271 186.

Il est connu de teindre les fibres kératiniques, et en particulier les cheveux humains, avec des compositions tinctoriales contenant des précurseurs de colorants d'oxydation et en particulier des p-phénylènediamines, des ortho- ou para-aminophénols appelés généralement "bases d'oxydation".

On sait également qu'on peut faire varier les nuances obtenues avec ces bases d'oxydation en les associant à des coupleurs encore appelés modificateurs de coloration comme les métadiamines aromatiques, les méta-aminophénols et les métadiphénols.

Le brevet US 4 013 404 décrit différents dérivés d'indole et leur utilisation en tant que colorants directs, coupleurs, ou précurseurs de colorants d'oxydation, suivant leur structure, pour la teinture des cheveux.

On recherche, dans le domaine de la teinture capillaire, des précurseurs de colorants d'oxydation ou des coupleurs permettant de conférer aux cheveux, en milieu alcalin oxydant généralement utilisé en teinture d'oxydation, une coloration ayant une résistance satisfaisante à la lumière, aux lavages, aux intempéries et à la transpiration.

La demande DE 30 31 709 a pour objet l'utilisation en tant que coupleur du 4-hydroxyindole en combinaison avec un précurseur de colorant d'oxydation, en présence d'un agent oxydant. Cette association ne présente toutefois pas de résultats satisfaisante quant aux propriétés des colorations qu'elle confère.

La damanderesse vient de découvrir, ce qui fait l'objet de l'invention, que certains dérivés du 4-hydroxyindole à titre de coupleurs, avec des précurseurs de colorants d'oxydation de type para ou ortho, permettaient d'obtenir, après application sur les fibres kératiniques et en particulier les cheveux, des teintures présentant des résistances à la lumière, aux lavages, aux intempéries et à la transpiration, particulièrement remarquables, notamment lorsque ces coupleurs sont utilisés avec la p-phénylànadiamine et ses dérivés.

Un objet de l'invention est donc constitué par des compositions tinctoriales d'oxydation, destinées à être utilisées pour la teinture des fibres kératiniques, contenant au moins un précurseur de colorant d'oxydation de type para et/ou ortho avec des dérivés de 4-hydroxyindole.

Un autre objet de l'invention est constitué par le procédé de coloration des fibres kératiniques, en particulier des cheveux humains, mettant en oeuvre une telle composition.

D'autres objets de l'invention apparaîtront à la lecture de la description et des examples qui suivent.

La composition tinctoriale d'oxydation conforme à l'invention, destinée à être utilisée pour la teinture des fibres kératiniques et en particulier des cheveux humains, est essentiellement caractérisée par le fait qu'elle contient dans un milieu approprié pour la teinture, au moins un précurseur de colorant d'oxydation para et/ou ortho et au moins un coupleur hétérocyclique, répondant à la formule (I) :

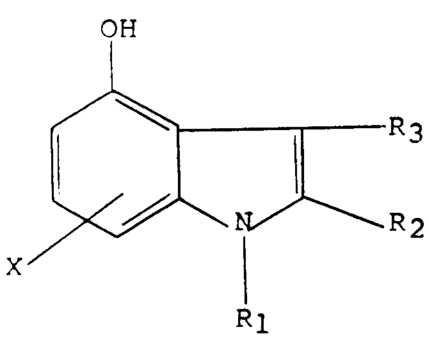

(I)

dans laquelle $R_1$ désigne un atome d'hydrogène, un radical alkyle en $C_1$-$C_4$; $R_2$ désigne un atome d'hydrogène, un radical alkyle inférieur en $C_1$-$C_4$, un radical carboxyle ou un radical alcoxycarbonyle; $R_3$ désigne un atome d'hydrogène ou d'halogène, un radical alkyle inférieur en $C_1$-$C_4$, un radical carboxyle, un radical alcoxycarbonyle ou un radical formyle; X désigne un atome d'hydrogène, un radical alkyle en $C_1$-$C_4$, un atome d'halogène, un radical alkoxy en $C_1$-$C_4$, un radical acétylamino, ou dialkyle ($C_1$-$C_4$) aminométhyle; au moins l'un des groupements X, $R_1$, $R_2$, $R_3$ étant différent de l'hydrogène; ainsi que leurs sels.

Parmi les composés de formule (I), les composés particulièrement préférés sont ceux dans lesquels le radical alkyle désigne méthyle, éthyle; le radical alcoxycarbonyle désigne méthoxy- ou éthoxycarbonyle.

Parmi ces composés, on peut citer le 4-hydroxy 5-éthoxyindole, le 4-hydroxy 5-méthoxyindole, le 4-hydroxy 1-méthyl 5-éthoxyindole, le 4-hydroxy 2-éthoxycarbonyl 5-éthoxyindole, le 4-hydroxy 2-méthyl 5-éthoxyindole, le 4-hydroxy 7-méthoxy 2,3-diméthylindole, le 4-hydroxy 5-méthylindole, le 4-hydroxy 3-chloroindole, le 4-hydroxy 5-diméthylaminométhylindole, le 4-hydroxy 3-formyl 2-méthyl 1-éthylindole, le 4-hydroxy 3-formylindole, le 4-hydroxy 3-formyl 2,6-diméthylindole, le 4-hydroxy 3-formyl 1-méthylindole, le 4-hydroxy 2-méthyl 5-éthoxyindole, le 4-hydroxy 1,5-diméthylindole, le 4-hydroxy 5-méthyl 2-carboxylindole, le 4-hydroxy 6-méthyl 3-éthoxy carbonylindole, le 4-hydroxy 7-méthylindole, le 4-hydroxy 2,5-diméthyl l'éthylindole, le 4-hydroxy 2,6-diméthyl 1-éthylindole, le 4-hydroxy 2-méthyl 1-éthylindole, le 4-hydroxy 3-éthylindole, le 4-hydroxy 2,6-diméthylindole, le 4-hydroxy 6-méthylindole, le 4-hydroxy 2-carboxy 5-méthylindole.

Le 4-hydroxy 1,5-diméthylindole et le 4-hydroxy 2-carboxy 5-méthylindole sont nouveaux et constituent un autre objet de l'invention.

Les précurseurs de colorants de type para ou ortho sont des composés qui ne sont pas des colorants en eux-mêmes, mais qui forment un colorant par un processus de condensation oxydative, soit sur eux-mêmes, soit en présence d'un coupleur ou modificateur.

Ces composés comportent des groupements fonctionnels, soit deux amino, soit un amino et un hydroxy, en position para ou ortho, l'un par rapport à l'autre.

Les précurseurs de type para sont choisis parmi les paraphénylènediamines, les para-aminophénols, les précurseurs hétérocycliques para, comme la 2,5-diaminopyridine, la 2-hydroxy 5-aminopyridine, la tétraaminopyrimidine, les bases dites "doubles".

A titre de paraphénylènediamines, on peut citer les composés répondant à la formule (II) ci-après :

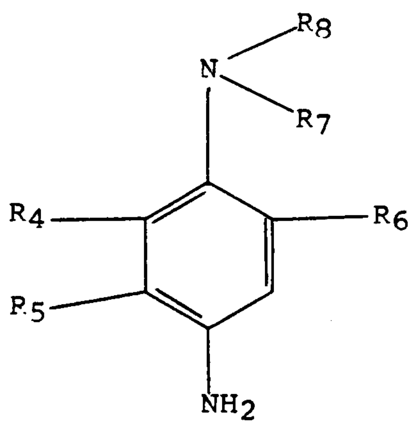

(II)

dans laquelle $R_4$, $R_5$ et $R_6$, identiques ou différents, représentent un atome d'hydrogène ou d'halogène, un radical alkyle ayant 1 à 4 atomes de carbone, un radical alcoxy ayant 1 à 4 atomes de carbone, $R_7$ et $R_8$, identiques ou différents, représentent un atome d'hydrogène, un radical alkyle, hydroxyalkyle, alkoxyalkyle, carbamylalkyle, mésylaminoalkyle, acétylaminoalkyle, uréidoalkyle, carbalkoxyaminoalkyle, pipéridinoalkyle, morpholinoalkyle, ces groupes alkyle ou alkoxy ayant de 1 à 4 atomes de carbone, ou bien $R_7$ et $R_8$ forment, conjointement avec l'atome d'azote auquel ils sont liés, un hétérocycle pipéridino ou morpholino, sous réserve que $R_4$ ou $R_6$ représente un atome d'hydrogène lorsque $R_7$ et $R_8$ ne représentent pas un atome d'hydrogène, ainsi que les sels de ces composés.

Parmi les composés de formule (II), on peut plus particulièrement citer la p-phénylènediamine, la p-toluylènediamine, la méthoxyparaphénylènediamine, la chloroparaphénylènediamine, la 2,6-diméthylparaphénylènediamine, la 2,5-diméthylparaphénylènediamine, la 2-méthyl 5-méthoxyparaphénylènediamine, la 2,6-diméthyl 5-méthoxyparaphénylènediamine, la N,N-diméthylparaphénylènediamine, la 3-méthyl 4-amino N,N-diéthylaniline, la N,N-di(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl,carbamylméthyl)aniline, la 3-méthyl 4-amino N,N-(éthyl,carbamylméthyl)aniline, la 4-amino N,N,-(éthyl,β-pipéridinoéthyl)aniline, la 3-méthyl 4-amino N,N-(éthyl,β-pipéridinoéthyl)aniline, la 4-amino N,N-(éthyl,β-morpholinoéthyl)aniline, la 3-méthyl 4-amino N,N-(éthyl,β-morpholinoéthyl)aniline, la 4-amino N,N-(éthyl,β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-(éthyl,β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3-méthyl 4-amino N,N-(éthyl,β-sulfoéthyl)aniline, la N-[(4'-amino)phényl] morpholine, la N-[(4'-amino)phényl]pipéridine.

Ces précurseurs de colorants par oxydation de type para peuvent être introduits dans la composition tinctoriale, soit sous forme de base libre, soit sous forme de sels, tels que sous forme de chlorhydrate, de bromhydrate ou de sulfate.

Parmi les p-aminophénols, on peut citer le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-(β-hydroxyéthyl)-4-aminophénol, le 2-méthoxy 4-aminophénol, le 3-méthoxy 4-aminophénol, le 2,5-diméthyl 4-aminophénol, le 2-méthoxyméthyl 4-aminophénol.

Les précurseurs de colorants d'oxydation de type ortho sont choisis parmi les orthoaminophénols, comme le 1-amino 2-hydroxybenzène, le 6-méthyl 1-hydroxy 2-aminobenzène, le 4-méthyl 1-amino 2-hydroxybenzène et les orthophénylènediamines.

Les bases dites doubles sont des bis-phénylalkylènediamines, répondant à la formule :

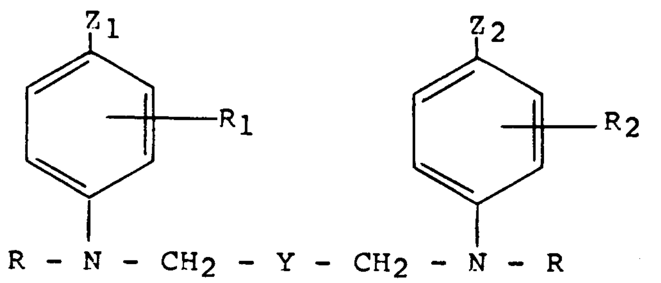

(IIbis)

dans laquelle :

$Z_1$ et $Z_2$, identiques ou différents, représentent des groupements hydroxyle ou $NHR_3$, où $R_3$ désigne un atome d'hydrogène ou un radical alcoyle inférieur;

$R_1$ et $R_2$, identiques ou différents, représentent soit des atomes d'hydrogènes, soit des atomes d'halogène, soit encore des groupements alcoyle;

R représente un atome d'hydrogène, un groupe alcoyle, hydroxyalcoyle ou aminoalcoyle, dont le reste amino peut être substitué;

Y représente un radical pris dans le groupe constitué par les radicaux suivants : $-(CH_2)_n-$, $(CH_2)_{n'}$, $-O-(CH_2)_{n'}-$, $-(CH_2)_{n'}-CHOH-(CH_2)_{n'}-(CH_2)_{n'}$,

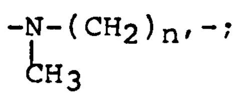

n étant un nombre entier compris entre 0 et 8 et n' un nombre entier compris entre 0 et 4, cette base pouvant se présenter sous forme de ses sels d'addition avec des acides.

Les radicaux alkyle ou alcoxy désignent de préférence un groupement ayant 1 à 4 atomes de carbone et notamment méthyle, éthyle, propyle, méthoxy, éthoxy.

Parmi les composés de formule (IIbis), on peut citer le N,N'bis-(β-hydroxyéthyl)N,N'-bis(4'-amino phényl)1,3-diamino 2-propanol, la N,N'bis-(β-hydroxyéthyl)N,N'-bis(4'-aminophényl)éthylènediamine, la N,N'bis-(4-aminophényl)tétraméthylènediamine, la N,N'bis(β-hydroxyéthyl)N,N'bis(4-aminophényl)-tétraméthylène diamine, la N,N'bis-(4-méthylaminophènyl)tétraméthylène diamine, la N,N'bis(éthyl)N,N'-bis-(4'-amino 3'-méthylphényl)éthylènediamine.

Les compositions tinctoriales peuvent également contenir en plus du coupleur hétérocyclique de la famille des 4-hydroxyindole de formule (I) définie ci-dessus, d'autres coupleurs connus en eux-mêmes, tels que les métadiphénols, les métaaminophénols, les métaphénylènediamines, les métaacylaminophénols, les métauréidophénols, les métacarbalcoxyaminophénols, l' α-naphtol, le 4-hydroxyindole, les coupleurs possédant un groupement méthylène actif, tels que les composés β-cétoniques et les pyrazolones.

Parmi ces coupleurs, on peut plus particulièrement citer le 2,4-dihydroxyphénoxyéthanol, le 2,4-dihydroxyanisole, le métaaminophénol, le monométhyléther de résorcine, le 2-méthyl 5-N-(β-hydroxyéthyl)-aminophénol, le 2-méthyl 5-N-(β-mésylaminoéthyl)aminophénol, la 6-hydroxybenzomorpholine, le 2,4-diaminoanisole, le 2,4-diaminophénoxyéthanol, la 6-aminobenzomorpholine, le [2-N-(β-hydroxyéthyl)amino 4-amino]-phénoxyéthanol, le 2-amino 4-N-(β-hydroxyéthyl) amino anisole, le (2,4-diamino)phényl-β, γ-dihydroxypropyléther, la 2,4-diaminophénoxyéthylamine, le 2-méthyl 5-aminophénol, le 2,6-diméthyl 3-aminophénol et leurs sels.

On peut rajouter à ces compositions, comme cela est bien connu dans l'état de la technique, notamment en vue de nuancer ou d'enricher en reflets les colorations apportées par les précurseurs de colorants d'oxydation et le coupleur de formule (I), des colorants directs, tels que des colorants azoïques, anthraquinoniques ou les dérivés nitrés de la série benzénique.

Ces compositions ne contiennent pas de dérivé quinonique de la famille des benzoquinones ou des naphtoquinones, susceptible d'oxyder le dérivé de 4-hydroxyindole.

Les compositions ne contiennent ni ion iodure ou nitrite présent dans ses quantités susceptibles d'oxyder les précurseurs de colorants d'oxydation et le coupleur hétérocyclique. Cette exclusion n'exclut pas la possibilité d'utiliser de l'eau contenant à l'état naturel ce type d'ions sous forme de traces.

L'ensemble des précurseurs de colorants par oxydation de type para et/ou ortho, ainsi que les coupleurs utilisés dans les compositions tinctoriales conformes à l'invention, représente de préférence de 0,3 à 7% en poids par rapport au poids de ladite composition. La concentration en composés (I) peut varier entre 0,05 et 3,5% en poids par rapport au poids total de la composition.

Le milieu approprié pour la teinture est constitué généralement par un milieu aqueux et son pH peut varier entre 8 et 11, et il est de préférence compris entre 9 et 11.

Il est ajusté à la valeur désirée à l'aide d'un agent alcalinisant, tel que l'ammoniaque, les carbonates alcalins, les alcanolamines comme la mono-, la di- ou la triéthanolamine.

Les compositions tinctoriales conformes à l'invention contiennent également, dans leur forme de réalisation préférée, des agents tensio-actifs anioniques, cationiques, non-ioniques, amphotères ou leurs mélanges. Parmi ces agents tensio-actifs, on peut citer les alkylbenzènesulfonates, les alkylnaphtalènesulfonates, les sulfates, les éthersulfates et les sulfonates d'alcools gras, les sels d'ammonium quaternaires, tels que le bromure de triméthylcétylammonium, le bromure de cétylpyridinium; les éthanolamides d'acides gras éventuellement oxyéthylénés; les acides, les alcools ou les amines polyoxyéthylénés, les alcools polyglycérolés, les alkylphénols polyoxyéthylénés ou polyglycérolés, ainsi que les alkylsulfates polyoxyéthylénés.

Ces agents tensio-actifs sont présents dans les compositions conformes à l'invention dans des proportions comprises entre 0,5 et 55% en poids, et de préférence entre 2 et 50% en poids par rapport au poids total de la composition.

Ces compositions peuvent également contenir des solvants organiques pour solubiliser les composés qui ne seraient pas suffisamment solubles dans l'eau. Parmi ces solvants, on peut citer à titre d'exemple, les alcanols inférieurs en $C_1$-$C_4$, tels que l'éthanol et l'isopropanol; le glycérol; les glycols ou éthers de glycol, comme le 2-butoxyéthanol, l'éthylèneglycol, le propylèneglycol, le monoéthyléther et le monométhyléther du diéthylèneglycol, ainsi que les alcools aromatiques comme l'alcool benzylique ou le phénoxyéthanol et les produits analogues ou leurs mélanges.

Les solvants sont présents de préférence dans des proportions comprises entre 1 et 40% en poids, et en particulier entre 5 et 30% en poids par rapport au poids total de la composition.

Les agents épaississants que l'on peut ajouter dans les compositions conformes à l'invention peuvent être choisis en particulier, parmi l'alginate de sodium, la gomme arabique, les dérivés de cellulose, les polymères d'acide acrylique, la gomme de xanthane. On peut également utiliser des agents épaississants minéraux, tels que la bentonite.

Ces agents épaississants sont présents de préférence dans des proportions comprises entre 0,1 et 5%, et en particulier entre 0,2 et 3% en poids par rapport au poids total de la composition.

Les agents antioxydants qui peuvent être présents dans les compositions sont choisis en particulier parmi le sulfite de sodium, l'acide thioglycolique, le bisulfite de sodium, l'acide ascorbique, l'hydroquinone et l'acide homogentisique. Ces agents antioxydants sont présents dans la composition dans des proportions comprises entre 0,05 et 1,5% en poids par rapport au poids total de la composition.

Ces compositions peuvent également contenir d'autres adjuvants cosmétiquement acceptables, tels que par exemple des agents de pénétration, des agents séquestrants, des parfums, des tampons, etc.

Les compositions conformes à l'invention peuvent se présenter sous des formes diverses, telles que sous forme de liquides, de crèmes, de gels ou sous toute autre forme appropriée pour réaliser une teinture des fibres kératiniques et notamment des cheveux humains. Ces compositions peuvent être conditionnées en flacons aérosols en présence d'un agent propulseur.

Les compositions tinctoriales conformes à l'invention contenant un précurseur de colorant par oxydation du type para et/ou ortho et un coupleur de formule (I), sont utilisées dans les procédés de teinture des fibres kératiniques et en particulier des cheveux humains, suivant un procédé mettant en oeuvre la révélation par un agent oxydant.

Conformément à ce procédé, on mélange au moment de l'emploi la composition tinctoriale décrite ci-dessus avec une solution oxydante en une quantité suffisante pour pouvoir développer une coloration, puis on applique le mélange obtenu sur les fibres kératiniques et en particulier, les cheveux humains.

La solution oxydante contient à titre d'agent oxydant l'eau oxygénés, la peroxyde d'urée ou des persels, tels que le persulfate d'ammonium. On utilise de préférence une solution d'eau oxygénée à 20 volumes.

Le mélange obtenu est appliqué sur les cheveux et on laisse poser pendant 10 à 40 minutes, de préférence 15 à 30 minutes, après quoi on rince les cheveux, on les lave au shampooing, on les rince à nouveau et on les sèche.

Le coupleur hétérocyclique de formule (I) définis ci-dessus peut également être mis en oeuvre dans un procédé à plusieurs étapes, consistant dans l'une des étapes, à appliquer le précurseur de colorant d'oxydation para et/ou ortho et, dans une autre étape à appliquer le coupleur de formule (I).

L'agent oxydant peut être introduit, tout juste avant l'application, dans la composition appliquée dans le deuxième temps ou bien être additionné sur les fibres kératiniques elles-mêmes dans un troisième temps, les conditions de pose et de séchage ou de lavage étant identiques.

Les exemples qui suivent sont destinés à illustrer l'invention.

Dans les exemples, on prépare la composition suivante :

COMPOSITION A

| | | |
|---|---|---|
| . Colorants | | x g |
| . Octyldodécanol vendu sous la dénomination d'EUTANOL G par la Société HENKEL | | 8,0 g |
| . Acide oléique | | 20,0 g |
| . Lauryl éther sulfate de monoéthanolamine vendu sous la dénomination de SIPON LM 35 par la Société HENKEL | | 3,0 g |
| . Alcool éthylique | | 10,0 g |
| . Alcool benzylique | | 10,0 g |
| . Alcool cétylstéarylique à 33 moles d'oxyde d'éthylène vendu sous la dénomination de SIMULSOL GS par la Société SEPPIC | | 2,4 g |
| . Acide éthylène diamine tétracétique | | 0,2 g |
| . Polymère cationique constitué de motifs récurrents : | | 2,2 g |

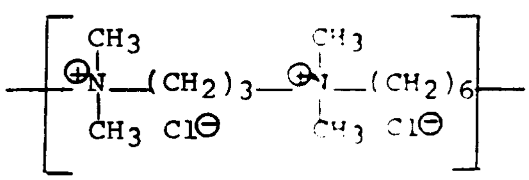

| | | |
|---|---|---|
| . Monoéthanolamine | | 7,5 g |
| . Diéthanolamide d'acide linoléique vendu sous la dénomination de COMPERLAN F par la Société HENKEL | | 8,0 g |
| . Ammoniaque à 20% de $NH_3$ | | 10,2 g |
| . Métabisulfite de sodium en solution aqueuse à 35% | | 1,3 g |
| . Hydroquinone | | 0,15 g |
| . 1-phényl 3-méthyl 5-pyrazolone | | 0,20 g |
| . Eau déminéralisée | qsp | 100,0 g |

Les colorants indiqués dans le tableau qui suit sont introduits dans les quantités mentionnées.

Ces compositions sont mélangées, respectivement, poids pour poids avec une composition oxydante titrant à 20 volumes en eau oxygénée et dont le pH est de 3.

Les mélanges ainsi réalisés sont appliqués 30 minutes sur des cheveux gris à 90% de blancs, puis rincés et lavés au shampooing et rincés à nouveau, puis séchés.

Les colorations qui figurent dans le tableau sont celles constatées après séchage.

COMPOSITION B

On prépare le mélange tinctorial suivant :

| | |
|---|---|
| . Colorants | x g |
| . Alcool oléique polyglycérolé à 2 moles de glycérol | 4,5 g |
| . Alcool oléique polyglycérolé à 4 moles de glycérol | 4,5 g |
| . ETHOMEEN O 12 - Société ARMOON HESS CHEMICAL Ltd (oléylamine oxyéthylénée à 12 moles d'oxyde d'éthylène) | 4,5 g |
| . COMPERLAN KD - Société HENKEL (diéthanolamide de coprah) | 9,0 g |
| . Propylèneglycol | 4,0 g |
| . 2-butoxyéthanol | 8,0 g |
| . Ethanol à 96° | 6,0 g |
| . MASQUOL DTPA - Société PROTEX (sel pentasodique de l'acide diéthylène triamine pentacétique) | 2,0 g |
| . Hydroquinone | 0,15 g |
| . Solution de bisulfite de sodium à 35° Bé | 1,3 g |
| . Ammoniaque à 22° Bé | 10,0 g |
| . Eau | qsp 100,0 g |

Les colorants indiqués dans le tableau qui suit sont introduits dans les quantités mentionnées.

Dans les exemples 1 à 10, on utilise la composition A.

Dans les exemples 11 à 17, on utilise la composition B.

Ces compositions sont mélangées, respectivement, poids pour poids avec une composition oxydante titrant à 20 volumes en eau oxygénée et dont le pH est de 3.

Les mélanges ainsi réalisés sont appliqués 30 minutes sur des cheveux gris à 90% de blancs pour les exemples 1 à 13 et 15 à 17, et sur des cheveux décolorés pour l'exemple 14, puis rincés et lavés au shampooing et rincés à nouveau, puis séchés.

Les colorations qui figurent dans le tableau sont celles constatées après séchage.

TABLEAU I

| Exemples | Coupleur hétérocyclique de formule (I) | g | Précurseur para | g | Couleur |
|---|---|---|---|---|---|
| 1 | 2,3-diméthyl 4-hydroxy 7-méthoxyindole | 0,382 | p-phénylènediamine | 0,216 | blond clair cendré beige |
| 2 | 2,3-diméthyl 4-hydroxy 7-méthoxyindole | 0,764 | p-aminophénol | 0,436 | blond irisé doré |
| 3 | 4-hydroxy 5-méthoxy-indole | 0,652 | p-aminophénol | 0,436 | blond beige cendré |
| 4 | 4-hydroxy 5-méthyl-indole | 0,32 | p-phénylènediamine | 0,216 | violine |
| 5 | 4-hydroxy 5-méthyl-indole | 0,65 | p-aminophénol | 0,436 | rouge cuivré |
| 6 | 4-hydroxy 5-méthoxy 2-méthylindole | 0,32 | p-phénylènediamine | 0,216 | blond clair cendré |
| 7 | 4-hydroxy 5-éthoxy 2-méthylindole | 0,75 | p-aminophénol | 0,436 | blond cendré |

TABLEAU II

| Exemples | Coupleur hétérocyclique de formule (I) | g | Précurseur para | g | Couleur |
|---|---|---|---|---|---|
| 8 | 4-hydroxy 5-éthoxy 2-éthoxycarbonylindole | 0,354 | p-phénylènediamine | 0,216 | blond clair irisé cendré |
| 9 | 4-hydroxy 5-éthoxy 2-éthoxycarbonylindole | 0,708 | p-aminophénol | 0,436 | blond clair cuivré irisé |
| 10 | 4-hydroxy 2-méthyl 5-éthoxyindole | 0,76 | p-aminophénol | 0,436 | blond clair beige doré |
| 11 | 1-méthyl 5-éthoxy 4-hydroxyindole | 0,478 | p-aminophénol | 0,272 | beige orangé |
| 12 | 4-hydroxy 5-méthylindole | 0,368 | p-aminophénol | 0,272 | orangé |
| 13 | 4-hydroxy 5-méthylindole | 0,368 | p-phénylènediamine | 0,270 | châtain pourpre |
| 14 | 4-hydroxy 5-(diméthyl-aminométhyl)indole | 0,475 | p-phénylènediamine | 0,270 | noir bleuté |
| 15 | 4-hydroxy 3-formylindole | 0,403 | p-phénylènediamine | 0,270 | châtain cendré |
| 16 | 4-hydroxy 3-formylindole | 0,403 | p-aminophénol | 0,272 | beige légèrement doré |
| 17 | 4-hydroxy 5-méthyl 2-carboxylindole | 0,478 | p-phénylènediamine | 0,270 | gris pourpre |

EXEMPLES DE PREPARATION

Exemple 1 : Préparation du 1,5-diméthyl 4-hydroxyindole

a) préparation du 4-benzyloxy 1,5-diméthylindole

On introduit successivement le 4-benzyloxy 5-méthylindole obtenu selon F. TROXLER et al, Helvetica Chimica Acta, 51, 1203 (1968) (2,5g, 0,0105 mole), de la soude à 50% (12ml), du toluène (7ml) et de l'hydrogéno sulfate de tétrabutyl ammonium (280mg).

On chauffe à 60 °C et on introduit le diméthylsulfate (1,2ml). On laisse à 60 °C pendant 30 minutes, on dilue à l'eau et on sépare les deux phases.

On lave la phase organique à l'eau, on la sèche et on évapore le solvant.

On obtient une huile vert pâle. Après chromatographie sur silice (éluant : $CH_2Cl_2$/heptane 50:50), on obtient une huile incolore de 4-benzyloxy 1,5-diméthylindole (1,9g)

Rendement = 72%.

| Analyse : $C_{17}H_{17}NO$ | | | | |
|---|---|---|---|---|
| | C | H | N | O |
| Calculé<br>Trouvé | 81,24<br>81,06 | 6,82<br>6,88 | 5,57<br>5,45 | 6,37<br>6,28 |

b) préparation du 1,5-diméthyl 4-hydroxyindole

Un mélange du dérivé précédent (1,8g, 0,0072mole), de cyclohexène (2,5ml), d'éthanol absolu (15ml) et de palladium sur charbon (0,4g) est porté au reflux pendant 3 heures.

On filtre à chaud et on évapore le solvant.

On obtient après passage sur colonne de silice (éluant $CH_2Cl_2$) une poudre blanc cassé de 1,5-diméthyl 4-hydroxyindole (0,5g).

Rendement = 44%, PF = 68 °C.

| Analyse : $C_{10}H_{11}NO$ | | | | |
|---|---|---|---|---|
| | C | H | N | O |
| Calculé<br>Trouvé | 74,51<br>74,48 | 6,88<br>6,90 | 8,69<br>8,72 | 9,92<br>10,10 |

Exemple 2 : Préparation du 2-carboxy 4-hydroxy 5-méthylindole

On introduit successivement le 4-benzyloxy 2-carboxy 5-méthylindole obtenu selon F. TROXLER et al, Helvetica Chimica Acta, 51, 1203 (1968) (0,8g, 2.84 $10^{-3}$mole), du cyclohexène (1,5ml), de l'éthanol absolu (8ml) et du palladium sur charbon (0,2g).

On porte au reflux pendant 2 heures. On filtre à chaud , on rince copieusement au méthanol chaud et on évapore le solvant. On recristallise dans le méthanol pour obtenir une poudre blanc cassé de 2-carboxy 4-hydroxy 5-méthylindole (0,5g)

Rendement = 61%, PF = 262 °C.

| Analyse : $C_{10}H_9NO_3$ | | | | |
|---|---|---|---|---|
| | C | H | N | O |
| Calculé<br>Trouvé | 62,82<br>62,85 | 4,74<br>4,76 | 7,33<br>7,28 | 25,11<br>25,35 |

## Revendications

**1.** Composition tinctoriale pour fibres kérantiniques, en particulier pour cheveux humains, caractérisée par le fait qu'elle contient dans un milieu approprié pour la teinture de ces fibres, au moins un précurseur de colorant d'oxydation para ou au moins un précurseur de colorant d'oxydation ortho en association avec au moins un coupleur hétérocyclique, répondant à la formule :

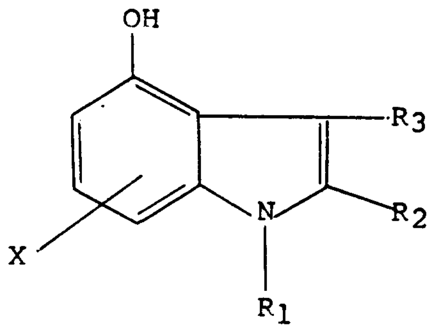

(I)

dans laquelle $R_1$ désigne un atome d'hydrogène, un radical alkyle en $C_1$-$C_4$; $R_2$ désigne un atome d'hydrogène, un radical alkyle inférieur en $C_1$-$C_4$, un radical carboxyle ou un radical alcoxycarbonyle; $R_3$ désigne un atome d'hydrogène ou d'halogène, un radical alkyle inférieur en $C_1$-$C_4$, un radical carboxyle, un radical alcoxycarbonyle ou un radical formyle; X désigne un atome d'hydrogène, un radical alkyle en $C_1$-$C_4$, un atome d'halogène, un radical alkoxy en $C_1$-$C_4$, un radical acétylamino ou dialkyle ($C_1$-$C_4$) aminométhyle;au moins l'un des groupements X, $R_1$, $R_2$, $R_3$ étant différent de l'hydrogène; ainsi que leurs sels.

**2.** Composition selon la revendication 1, caractérisée par le fait que les composés de formule (I) sont choisis parmi le 4-hydroxy 5-éthoxyindole, le 4-hydroxy 5-méthoxyindole, le 4-hydroxy 1-méthyl 5-éthoxyindole, le 4-hydroxy 2-éthoxycarbonyl 5-éthoxyindole, le 4-hydroxy 2-méthyl 5-éthoxyindole, le 4-hydroxy 7-méthoxy 2,3-diméthylindole, le 4-hydroxy 5-méthylindole le 4-hydroxy 3-chloroindole, le 4-hydroxy 5-diméthylamino méthylindole, le 4-hydroxy 3-formyl 2-méthyl 1-éthylindole, le 4-hydroxy 3-formylindole, le 4-hydroxy 3-formyl 2,6-diméthylindole, le 4-hydroxy 3-formyl 1-méthylindole, le 4-hydroxy 2-méthyl 5-éthoxy indole, le 4-hydroxy 1,5-diméthyl-indole, le 4-hydroxy 5-méthyl 2-carboxylindole, le 4-hydroxy 6-méthyl 3-éthoxycarbonylindole, le 4-hydroxy 7-méthylindole, le 4-hydroxy 2,5-diméthyl 1-éthylindole, le 4-hydroxy 2,6-diméthyl 1-éthylindole, le 4-hydroxy 2-méthyl 1-éthylindole, le 4-hydroxy 3-éthylindole, le 4-hydroxy 2,6-diméthylindole, le 4-hydroxy 6-méthylindole, le 4-hydroxy 2-carboxy 5-méthylindole.

**3.** Composition selon l'une quelconque des revendications 1 ou 2, caractérisée par le fait que les précurseurs de colorants d'oxydation para sont choisis parmi les paraphénylènediamines, les para-aminophénols, les précurseurs hétérocycliques para, les bases dites "doubles".

**4.** Composition selon l'une quelconque des revendications 1 à 3, caractérisée par le fait que les paraphénylènediamines sont choisis parmi les composés répondant à la formule (II) :

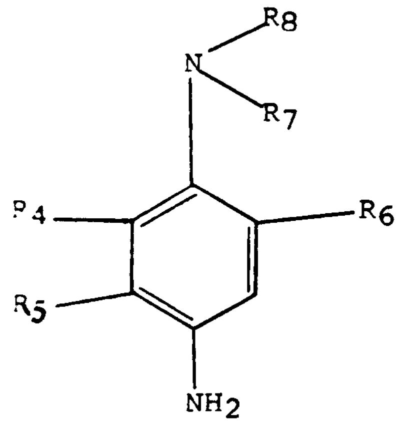

(II)

dans laquelle $R_4$, $R_5$ et $R_6$, identiques ou différents, représentent un atome d'hydrogène ou d'halogène, un radical alkyle ayant 1 à 4 atomes de carbone, un radical alcoxy ayant 1 à 4 atomes de carbone, $R_7$ et $R_8$, identiques ou différents, représentent un atome d'hydrogène, un radical alkyle, hydroxyalkyle, alkoxyalkyle, carbamylalkyle, mésylaminoalkyle, acétylaminoalkyle, uréidoalkyle, carbalkoxyaminoalkyle, pipéridinoalkyle, morpholinoalkyle, dans lesquels les groupements alkyle ou alkoxy ont de 1 à 4 atomes de carbone, ou bien $R_7$ et $R_8$ forment, conjointement avec l'atome d'azote auquel ils sont liés, un hétérocycle pipéridino ou morpholino, sous réserve que $R_4$ ou $R_6$ représente un atome d'hydrogène lorsque $R_7$ et $R_8$ ne représentent pas un atome d'hydrogène, ainsi que les sels de ces composés.

**5.** Composition selon la revendication 4, caractérisée par le fait que les composés de formule (II) sont choisis parmi la p-phénylènediamine, la p-toluylènediamine, la méthoxyparaphénylènediamine, la chloroparaphénylènediamine, la 2,6-diméthylparaphénylènediamine, la 2,5-diméthylparaphénylènediamine, la 2-méthyl 5-méthoxyparaphénylènediamine, la 2,6-diméthyl 5-méthoxyparaphénylènediamine, la N,N-diméthylparaphénylènediamine, la 3-méthyl 4-amino N,N-diéthylaniline, la N,N-di($\beta$-hydroxyéthyl)-paraphénylènediamine, la 3-méthyl 4-amino N,N-di-($\beta$-hydroxyéthyl)aniline, la 3-chloro 4-amino N,N-di-($\beta$-hydroxyéthyl)aniline, la 4-amino N,N-(éthyl,carbamylméthyl)aniline, la 3-méthyl 4-amino N,N-(éthyl,carbamylméthyl)aniline, la 4-amino N,N-(éthyl-$\beta$-pipéridinoéthyl)aniline, la 3-méthyl 4-amino N,N-(éthyl,$\beta$-pipéridinoéthyl)aniline, la 4-amino N,N-(éthyl,$\beta$-morpholinoéthyl)aniline, la 3-méthyl 4-amino N,N-(éthyl-$\beta$-morpholinoéthyl)aniline, la 4-amino N,N-(éthyl,$\beta$-acétylaminoéthyl)aniline, la 4-amino N-($\beta$-méthoxyéthyl)aniline, la 3-méthyl 4-amino N,N-(éthyl,$\beta$-acétylaminoéthyl)aniline, la 4-amino N,N-(éthyl,$\beta$-mésylaminoéthyl)aniline, la 3-méthyl 4-amino N,N-(éthyl,$\beta$-mésylaminoéthyl)aniline, la 4-amino N,N-(éthyl,$\beta$-sulfoéthyl)aniline, la 3-méthyl 4-amino N,N-(éthyl,$\beta$-sulfoéthyl)aniline, la N-[(4'-amino)-phényl] morpholine, la N-[(4'-amino)phényl]pipéridine et leurs sels.

**6.** Composition selon l'une quelconque des revendications 1 à 4, caractérisée par le fait que les p-aminophénols sont choisis parmi le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-($\beta$-hydroxyéthyl)-4-aminophénol, le 2-méthoxy 4-aminophénol, le 3-méthoxy 4-aminophénol, le 2,5-diméthyl 4-aminophénol, le 2-méthoxyméthyl 4-aminophénol.

**7.** Composition selon la revendication 1 ou 2, caractérisée par le fait que les précurseurs de colorants d'oxydation de type ortho sont choisis parmi les orthoaminophénols et les orthophénylènediamines.

**8.** Composition selon la revendication 3, caractérisée par le fait que les bases doubles sont des bis-phénylalkylènediamines répondant à la formule :

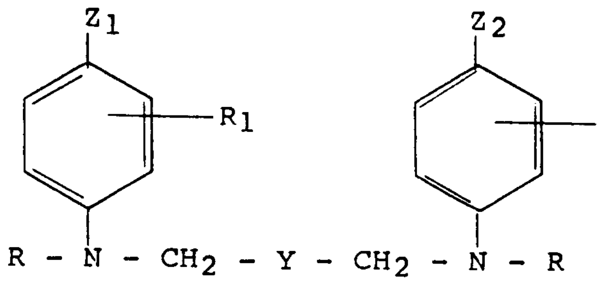

(IIbis)

dans laquelle :

$Z_1$ et $Z_2$, identiques ou différents, représentent des groupement hydroxyle ou $NHR_3$, où $R_3$ désigne un atome d'hydrogène ou un radical alcoyle inférieur = $C_1$-$C_4$;

$R_1$ et $R_2$, identiques ou différents, représentent soit des atomes d'hydrogène, soit des atomes d'halogène, soit encore des groupements alcoyle;

R représente un atome d'hydrogène, un groupe alcoyle, hydroxyalcoyle ou aminoalcoyle, dont le reste amino peut être substitué;

Y représente un radical pris dans le groupe constitué par les radicaux suivants : $-(CH_2)_n-$, $(CH_2)_n$, $-O-(CH_2)_{n'}-$, $-(CH_2)_{n'}-CHOH-(CH_2)_{n'}-(CH_2)_n$,

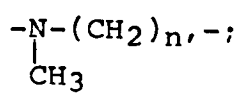

n étant un nombre entier compris entre 0 et 8 et n' un nombre entier compris entre 0 et 4, cette base pouvant se présenter sous forme de ses sels d'addition avec des acides.

**9.** Composition selon la revendication 8, caractérisée par le fait que les composés de formule (IIbis) sont choisis parmi le N,N'bis-($\beta$-hydroxyéthyl) N,N'-bis(4'-aminophényl)1,3-diamino 2-propanol, la N,N'bis-($\beta$-hydroxyéthyl)N,N'-bis-(4'-aminophényl)éthyl ènediamine, la N,N'bis-(4-aminophényl)tétraméthylène diamine, la N,N'bis-($\beta$-hydroxyéthyl)N,N'bis-(4-amino phényl)tétraméthylènediamine, la N,N'bis-(4-méthyl aminophényl)tétraméthylènediamine, la N,N'bis-(éthyl) N,N'-bis(4'-amino 3'-méthylphényl)éthylène diamine.

**10.** Composition selon l'une quelconque des revendications 1 à 9, caractérisée par le fait que la composition contient également en plus des coupleurs hétérocycliques de formule (I), des métadiphénols, des métaaminophénols, des métaphénylènediamines, des métaacylaminophénols, des métauréidophénols, des métacarbalcoxyaminophénols, l' $\alpha$-naphtol, le 4-hydroxy indole, des composés $\beta$-cétoniques et des pyrazolones.

**11.** Composition selon l'une quelconque des revendications 1 à 10, caractérisée par le fait que la composition contient également des colorants directs.

**12.** Composition selon l'une quelconque des revendications 1 à 11, caractérisée par le fait que les colorants par oxydation de type para et/ou ortho et les coupleurs sont présents dans des proportions comprises entre 0,3 et 7% en poids par rapport au poids total de la composition.

**13.** Composition selon l'une quelconque des revendications 1 à 12, caractérisée par le fait que le composé de formule (I) est présent dans des proportions comprises entre 0,05 et 3,5% en poids par rapport au poids total de la composition.

**14.** Composition selon l'une quelconque des revendications 1 à 13, caractérisée par le fait que le milieu approprié pour la teinture de ces fibres est aqueux et qu'il a un pH compris entre 8 et 11.

**15.** Composition selon l'une quelconque des revendications 1 à 14, caractérisée par le fait que la composition contient également des agents tensio-actifs anioniques, cationiques, non ioniques, amphotères ou leurs mélanges.

**16.** Composition selon l'une quelconque des revendications 1 à 15, caractérisée par le fait que la composition contient également des solvants organiques dans des proportions comprises entre 1 et 40% en poids par rapport au poids total de la composition.

**17.** Composition selon l'une quelconque des revendications 1 à 16, caractérisée par le fait que la composition contient également des agents épaississants, des agents antioxydants, des agents de pénétration, des agents séquestrants, des tampons et/ou des parfums.

**18.** Composition selon l'une quelconque des revendications 1 à 17, caractérisée par le fait qu'elle se présente sous forme de liquides, de crèmes, de gels et qu'elle est éventuellement conditionnée en aérosols en présence d'un agent propulseur.

**19.** Procédé de teinture des fibres kératiniques, en particulier des cheveux humains, caractérisé par le fait que l'on applique une composition telle que définie dans l'une quelconque des revendications 1 à 18, sur lesdites fibres, en présence d'une solution oxydante, qu'on laisse la composition au contact des fibres pendant 10 à 40 minutes, qu'on fait suivre l'application par un rinçage de cheveux, un lavage, un rinçage et un séchage.

**20.** Procédé de teinture des fibres kératiniques, en particulier des cheveux humains, caractérisé par le fait que l'on applique dans un premier temps, le précurseur du colorant d'oxydation para et/ou ortho et, dans un second temps, au moins un coupleur hétérocyclique de formule (I), l'agent oxydant étant, soit introduit tout juste avant l'emploi dans la composition appliquée dans le second temps, ou bien additionné sur les fibres dans un troisième temps.

**21.** Utilisation d'un composé répondant à la formule :

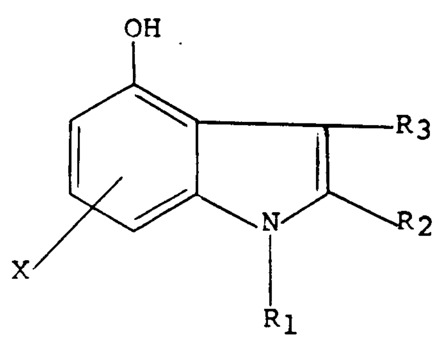

(I)

dans laquelle $R_1$ désigne un atome d'hydrogène, un radical alkyle en $C_1$-$C_4$; $R_2$ désigne un atome d'hydrogène, un radical alkyle inférieur en $C_1$-$C_4$, un radical carboxyle ou un radical alcoxycarbonyle; $R_3$ désigne un atome d'hydrogène ou d'halogène, un radical alkyle inférieur en $C_1$-$C_4$, un radical carboxyle, un radical alcoxycarbonyle ou un radical formyle; X désigne un atome d'hydrogène, un radical alkyle en $C_1$-$C_4$, un atome d'halogène, un radical alkoxy en $C_1$-$C_4$, un groupement acétylamino ou dialkyle ($C_1$-$C_4$) aminoéthyle; au moins l'un des groupements X, $R_1$, $R_2$, $R_3$ étant différent de l'hydrogène; ainsi que leurs sels à titre de coupleur pour la teinture par oxydation des fibres kératiniques mettant en oeuvre au moins un précurseur de colorant d'oxydation para et/ou ortho.

**22.** Composé nouveau choisi parmi le 4-hydroxy 1,5-diméthylindole et le 4-hydroxy 2-carboxy 5-méthylindole.

## Claims

**1.** Tinctorial composition for keratinous fibres, in particular for human hair, characterized in that it contains, in a medium appropriate for dyeing these fibres, at least one para oxidation dye precursor or at least one ortho oxidation dye precursor in combination with at least one heterocyclic coupler, corresponding to the formula:

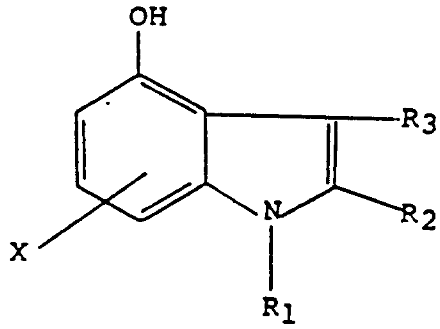

(I)

in which $R_1$ denotes a hydrogen atom or a $C_1$-$C_4$ alkyl radical; $R_2$ denotes a hydrogen atom, a $C_1$-$C_4$ lower alkyl radical, a carboxyl radical or an alkoxycarbonyl radical; $R_3$ denotes a hydrogen or halogen atom, a $C_1$-$C_4$ lower alkyl radical, a carboxyl radical, an alkoxycarbonyl radical or a formyl radical; X denotes a hydrogen atom, a $C_1$-$C_4$ alkyl radical, a halogen atom, a $C_1$-$C_4$ alkoxy radical, an acetylamino radical or a dialkyl($C_1$-$C_4$)aminomethyl radical; at least one of the groups X, $R_1$, $R_2$ and $R_3$ differing from hydrogen; as well as their salts.

2. Composition according to Claim 1, characterized in that the compounds of formula (I) are chosen from 4-hydroxy-5-ethoxyindole, 4-hydroxy-5-methoxyindole, 4-hydroxy-1-methyl-5-ethoxyindole, 4-hydroxy-2-ethoxycarbonyl-5-ethoxyindole, 4-hydroxy-2-methyl-5-ethoxyindole, 4-hydroxy-7-methoxy-2,3-dimethylindole, 4-hydroxy-5-methylindole, 4-hydroxy-3-chloroindole, 4-hydroxy-5-dimethylaminomethylindole, 4-hydroxy-3-formyl-2-methyl-1-ethylindole, 4-hydroxy-3-formylindole, 4-hydroxy-3-formyl-2,6-dimethylindole, 4-hydroxy-3-formyl-1-methylindole, 4-hydroxy-2-methyl-5-ethoxyindole, 4-hydroxy-1,5-dimethylindole, 4-hydroxy-5-methyl-2-carboxylindole, 4-hydroxy-6-methyl-3-ethoxycarbonylindole, 4-hydroxy-7-methylindole, 4-hydroxy-2,5-dimethyl-1-ethylindole, 4-hydroxy-2,6-dimethyl-1-ethylindole, 4-hydroxy-2-methyl-1-ethylindole, 4-hydroxy-3-ethylindole, 4-hydroxy-2,6-dimethylindole, 4-hydroxy-6-methylindole and 4-hydroxy-2-carboxy-5-methylindole.

3. Composition according to either of Claims 1 or 2, characterized in that the para oxidation dye precursors are chosen from paraphenylenediamines, para-aminophenols, para heterocyclic precursors and the so-called "double" bases.

4. Composition according to any one of Claims 1 to 3, characterized in that the paraphenylenediamines are chosen from the compounds corresponding to the formula (II):

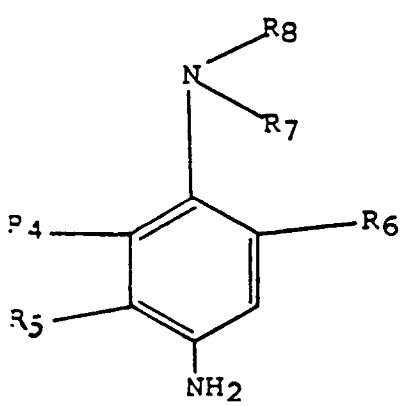

(II)

in which $R_4$, $R_5$ and $R_6$, which may be identical or different, represent a hydrogen or halogen atom, an alkyl radical having 1 to 4 carbon atoms or an alkoxy radical having 1 to 4 carbon atoms, and $R_7$ and $R_8$, which may be identical or different, represent a hydrogen atom or an alkyl, hydroxyalkyl, alkoxyalkyl, carbamylalkyl, mesylaminoalkyl, acetylaminoalkyl, ureidoalkyl, carbalkoxyaminoalkyl, piperidinoalkyl or morpholinoalkyl radical, in which the alkyl or alkoxy groups have from 1 to 4 carbon atoms, or $R_7$ and $R_8$ form, together with the nitrogen atom to which they are bonded, a piperidino or morpholino heterocycle, with the proviso that $R_4$ or $R_6$ represents a hydrogen atom when $R_7$ and $R_8$ do

not represent a hydrogen atom, as well as the salts of these compounds.

**5.** Composition according to Claim 4, characterized in that the compounds of formula (II) are chosen from p-phenylenediamine, p-toluylenediamine, methoxyparaphenylenediamine, chloroparaphenylenediamine, 2,6-dimethylparaphenylenediamine, 2,5-dimethylparaphenylenediamine, 2-methyl-5-methoxyparaphenylenediamine, 2,6-dimethyl-5-methoxyparaphenylenediamine, N,N-dimethylparaphenylenediamine,3-methyl-4-amino-N,N-diethylaniline, N,N-di(β-hydroxyethyl)-paraphenylenediamine, 3-methyl-4-amino-N,N-di-(β-hydroxyethyl)aniline, 3-chloro-4-amino-N,N-di-(β-hydroxyethyl)aniline, 4-amino-N,N-(ethyl,carbamylmethyl)aniline, 3-methyl-4-amino-N,N-(ethyl,carbamylmethyl)aniline, 4-amino-N,N-(ethyl,β-piperidinoethyl)aniline, 3-methyl-4-amino-N,N-(ethyl,β-piperidinoethyl)aniline, 4-amino-N,N-(ethyl,β-morpholinoethyl)aniline, 3-methyl-4-amino-N,N-(ethyl,β-morpholinoethyl)aniline, 4-amino-N,N-(ethyl,β-acetylaminoethyl)aniline, 4-amino-N-(β-methoxyethyl)aniline, 3-methyl-4-amino-N,N-(ethyl,β-acetylaminoethyl)aniline, 4-amino-N,N-(ethyl,β-mesylaminoethyl)aniline, 3-methyl-4-amino-N,N-(ethyl,β-mesylaminoethyl)aniline, 4-amino-N,N-(ethyl,β-sulphoethyl)aniline, 3-methyl-4-amino-N,N-(ethyl,β-sulphoethyl)aniline, N-[(4'-amino)phenyl]morpholine and N-[(4'-amino)phenyl]piperidine, and their salts.

**6.** Composition according to any one of Claims 1 to 4, characterized in that the p-aminophenols are chosen from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2-hydroxymethyl-4-aminophenol, 2-(β-hydroxyethyl)-4-aminophenol, 2-methoxy-4-aminophenol, 3-methoxy-4-aminophenol, 2,5-dimethyl-4-aminophenol and 2-methoxymethyl-4-aminophenol.

**7.** Composition according to Claim 1 or 2, characterized in that the oxidation dye precursors of ortho type are chosen from ortho-aminophenols and orthophenylenediamines.

**8.** Composition according to Claim 3, characterized in that the double bases are bis-phenylalkylenediamines corresponding to the formula:

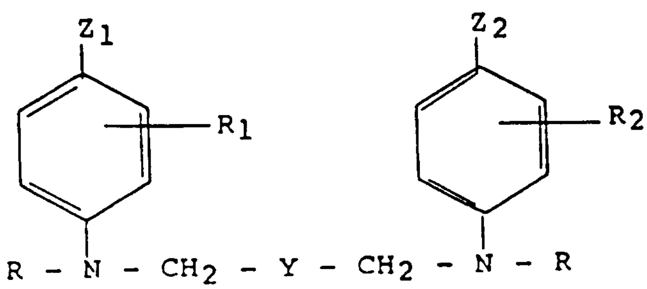

**(IIa)**

in which:

$Z_1$ and $Z_2$, which may be identical or different, represent hydroxyl or $NHR_3$ groups, where $R_3$ denotes a hydrogen atom or a $C_1$-$C_4$ lower alkyl radical;

$R_1$ and $R_2$, which may be identical or different, represent either hydrogen atoms or halogen atoms or alkyl groups;

R represents a hydrogen atom or an alkyl, hydroxyalkyl or aminoalkyl group, in which the amino radical can be substituted; and

Y represents a radical taken from the group comprising the following radicals: $-(CH_2)_n-$, $(CH_2)_n$, $-O-(CH_2)_{n'}-$, $-(CH_2)_{n'}-CHOH-(CH_2)_{n'}-(CH_2)_{n'}$,

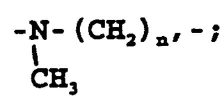

n being an integer between 0 and 8 and n' being an integer between 0 and 4, it being possible for this base to be in the form of its addition salts with acids.

**9.** Composition according to Claim 8, characterized in that the compounds of formula (IIa) are chosen from N,N'-bis(β-hydroxyethyl)-N,N'-bis(4'-aminophenyl)-1,3-diaminopropan-2-ol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)-tetramethylenediamine and N,N'-bis(ethyl)-N,N'-bis(4,-amino-3'-methylphenyl)ethylenediamine.

**10.** Composition according to any one of Claims 1 to 9, characterized in that the composition also contains, in addition to the heterocyclic couplers of formula (I), metadiphenols, meta-aminophenols, metaphenylenediamines, meta-acylaminophenols, meta-ureidophenols, metacarbalkoxyaminophenols, α-naphthol, 4-hydroxyindole, β-keto compounds and pyrazolones.

**11.** Composition according to any one of Claims 1 to 10, characterized in that the composition also contains direct dyes.

**12.** Composition according to any one of Claims 1 to 11, characterized in that the oxidation dyes of para and/or ortho type and the couplers are present in proportions of between 0.3 and 7% by weight relative to the total weight of the composition.

**13.** Composition according to any one of Claims 1 to 12, characterized in that the compound of formula (I) is present in proportions of between 0.05 and 3.5% by weight relative to the total weight of the composition.

**14.** Composition according to any one of Claims 1 to 13, characterized in that the medium appropriate for dyeing these fibres is aqueous and in that it has a pH of between 8 and 11.

**15.** Composition according to any one of Claims 1 to 14, characterized in that the composition also contains anionic, cationic, nonionic or amphoteric surfactants or their mixtures.

**16.** Composition according to any one of Claims 1 to 15, characterized in that the composition also contains organic solvents in proportions of between 1 and 40% by weight relative to the total weight of the composition.

**17.** Composition according to any one of Claims 1 to 16, characterized in that the composition also contains thickeners, antioxidants, penetration agents, sequestering agents, buffers and/or perfumes.

**18.** Composition according to any one of Claims 1 to 17, characterized in that it is in the form of liquids, creams or gels and in that it is optionally packaged as aerosols in the presence of a propellant.

**19.** Method for dyeing keratinous fibres, in particular human hair, characterized in that a composition as defined in any one of Claims 1 to 18 is applied to the said fibres in the presence of an oxidizing solution, in that the composition is left in contact with the fibres for 10 to 40 minutes and in that the application is followed by rinsing of the hair, washing, rinsing and drying.

**20.** Method for dyeing keratinous fibres, in particular human hair, characterized in that the para and/or ortho oxidation dye precursor is applied in a first step and at least one heterocyclic coupler of formula (I) is applied in a second step, the oxidizing agent either being introduced just before use into the composition applied in the second step, or being added to the fibres in a third step.

**21.** Use of a compound corresponding to the formula:

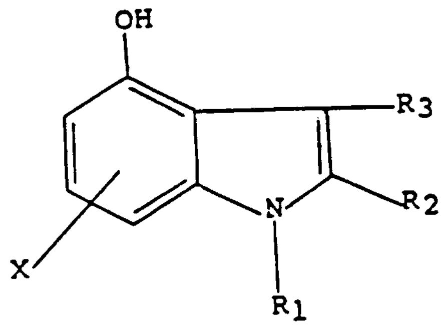

(I)

in which $R_1$ denotes a hydrogen atom or a $C_1$-$C_4$ alkyl radical; $R_2$ denotes a hydrogen atom, a $C_1$-$C_4$ lower alkyl radical, a carboxyl radical or an alkoxycarbonyl radical; $R_3$ denotes a hydrogen or halogen atom, a $C_1$-$C_4$ lower alkyl radical, a carboxyl radical, an alkoxycarbonyl radical or a formyl radical; X denotes a hydrogen atom, a $C_1$-$C_4$ alkyl radical, a halogen atom, a $C_1$-$C_4$ alkoxy radical, an acetylamino group or a dialkyl($C_1$-$C_4$)aminomethyl group; at least one of the groups X, $R_1$, $R_2$ and $R_3$ differing from hydrogen; as well as their salts, as coupler for oxidation dyeing of keratinous fibres using at least one para and/or ortho oxidation dye precursor.

**22.** New compound chosen from 4-hydroxy-1,5-dimethylindole and 4-hydroxy-2-carboxy-5-methylindole.

## Patentansprüche

**1.** Färbezusammensetzung für keratinische Fasern, insbesondere für menschliche Haare,
dadurch **gekennzeichnet**, daß
sie in einem zur Färbung dieser Fasern geeigneten Milieu mindestens eine Oxidationsfarbstoff-Vorstufe vom para-Typ oder mindestens eine Oxidationsfarbstoff-Vorstufe vom ortho-Typ zusammen mit mindestens einem heterozyklischen Kuppler der Formel:

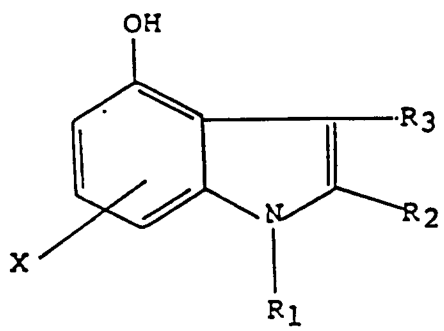

(I)

worin $R_1$ ein Wasserstoffatom, einen $C_{1-4}$-Alkylrest, $R_2$ ein Wasserstoffatom, einen $C_{1-4}$-Niedrigalkyl-, einen Carboxyl- oder Alkoxycarbonylrest, $R_3$ ein Wasserstoff- oder Halogenatom, einen $C_{1-4}$-Niedrigalkylrest, einen Carboxyl-, Alkoxycarbonyl- oder Formylrest und X ein Wasserstoffatom, einen $C_{1-4}$-Alkylrest, ein Halogenatom, einen $C_{1-4}$-Alkoxy-, einen Acetylamino- oder $C_{1-4}$-Dialkylaminomethylrest darstellen, wobei mindestens eine der Gruppen X, $R_1$, $R_2$, $R_3$ von Wasserstoff verschieden ist, sowie dessen Salze,
enthält.

**2.** Zusammensetzung gemäß Anspruch 1,
dadurch **gekennzeichnet**, daß
die Verbindungen der Formel (I) aus 4-Hydroxy-5-ethoxyindol, 4-Hydroxy-5-methoxyindol, 4-Hydroxy-1-methyl-5-ethoxyindol, 4-Hydroxy-2-ethoxycarbonyl-5-ethoxyindol, 4-Hydroxy-2-methyl-5-ethoxyindol, 4-Hydroxy-7-methoxy-2,4-dimethylindol, 4-Hydroxy-5-methylindol, 4-Hydroxy-3-chlorindol, 4-Hydroxy-5-dimethylaminomethylindol, 4-Hydroxy-3-formyl-2-methyl-1-ethylindol, 4-Hydroxy-3-formylindol, 4-Hydroxy-3-formyl-2,6-dimethylindol, 4-Hydroxy-3-formyl-1-methylindol, 4-Hydroxy-2-methyl-5-ethoxyindol, 4-Hydroxy-1,5-dimethylindol, 4-Hydroxy-5-methyl-2-carboxyindol, 4-Hydroxy-6-methyl-3-ethoxycarbonylindol, 4-Hydroxy-7-methylindol, 4-Hydroxy-2,5-dimethyl-1-ethylindol, 4-Hydroxy-2,6-dimethyl-1-ethylindol, 4-Hydroxy-2-methyl-1-ethylindol, 4-Hydroxy-3-ethylindol 4-Hydroxy-2,6-dimethylindol, 4-Hydroxy-6-methylindol und 4-Hydroxy-2-carboxy-5-methylindol
ausgewählt sind.

**3.** Zusammensetzung gemäß jedem der Ansprüche 1 oder 2,
dadurch **gekennzeichnet**, daß
die Oxidationsfarbstoff-Vorstufen vom para-Typ aus p-Phenylendiaminen, p-Aminophenolen, heterozyklischen Vorstufen vom para-Typ und aus den als "doppelte" bezeichneten Basen ausgewählt sind.

**4.** Zusammensetzung gemäß jedem der Ansprüche 1 bis 3,
dadurch **gekennzeichnet**, daß
die p-Phenylendiamine aus Verbindungen der Formel (II) ausgewählt sind:

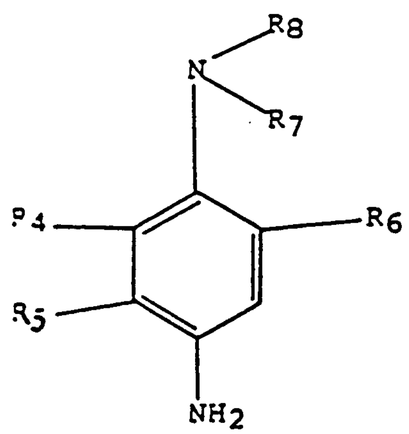

(II)

worin $R_4$, $R_5$ und $R_6$, gleich oder verschieden, ein Wasserstoff- oder Halogenatom, einen Alkylrest mit 1 bis 4 Kohlenstoffatomen, einen Alkoxyrest mit 1 bis 4 Kohlenstoffatomen, $R_7$ und $R_8$, gleich oder verschieden, ein Wasserstoffatom, einen Alkyl-, Hydroxyalkyl-, Alkoxyalkyl-, Carbamylalkyl-, Mesylaminoalkyl-, Acetylaminoalkyl-, Ureidoalkyl-, Carbalkoxyaminoalkyl-, Piperidinoalkyl-, Morpholinoalkylrest darstellen, wobei die Alkyl- oder Alkoxygruppen 1 bis 4 Kohlenstoffatome aufweisen, oder $R_7$ und $R_8$, zusammen mit dem Stickstoffatom, an das sie gebunden sind, einen Piperidino- oder Morpholino-Heterozyklus bilden, mit der Maßgabe, daß $R_4$ oder $R_6$ ein Wasserstoffatom darstellen, wenn $R_7$ und $R_8$ kein Wasserstoffatom darstellen, sowie aus Salzen dieser Verbindungen.

**5.** Zusammensetzung gemäß Anspruch 4,
dadurch **gekennzeichnet**, daß
die Verbindungen der Formel (II) aus p-Phenylendiamin, p-Toluylendiamin, Methoxy-p-phenylendiamin, Chlor-p-phenylendiamin, 2,6-Dimethyl-p-phenylendiamin, 2,5-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, 3-Methyl-4-amino-N,N-diethylanilin, N,N-Di(β-hydroxyethyl)-p-phenylendiamin, 3-Methyl-4-amino-N,N-di-(β-hydroxyethyl)anilin, 3-Chlor-4-amino-N,N-di-(β-hydroxyethyl)anilin, 4-Amino-N,N-(ethyl,carbamylmethyl)anilin, 3-Methyl-4-amino-N,N-(ethyl)carbamylmethyl)anilin, 4-Amino-N,N-(ethyl,β-piperidinoethyl)anilin, 3-Methyl-4-amino-N,N-(ethyl,β-piperidinoethyl)anilin, 4-Amino-N,N-(ethyl,β-morpholinoethyl)anilin, 3-Methyl-4-amino-N,N-(ethyl,β-morpholinoethyl)anilin, 4-Amino-N,N-(ethyl,β-acetylaminoethyl)anilin, 4-Amino-N-(β-methoxyethyl)anilin, 3-Methyl-4-amino-N,N-(ethyl,β-acetylaminoethyl)anilin, 4-Amino-N,N-(ethyl,β-mesylaminoethyl)anilin, 3-Methyl-4-amino-N,N-(ethyl,β-mesylaminoethyl)anilin, 4-Amino-N,N-(ethyl,β-sulfoethyl)anilin, 3-Methyl-4-amino-N,N-(ethyl,β-sulfoethyl)anilin, N-((4'-Amino)-phenyl)morpholin, N-((4'-Amino)phenyl)piperidin und aus deren Salzen ausgewählt sind.

**6.** Zusammensetzung gemäß jedem der Ansprüche 1 bis 4,
dadurch **gekennzeichnet**, daß
die p-Aminophenole aus p-Aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2-Hydroxymethyl-4-aminophenol, 2-(β-Hydroxyethyl)-4-aminophenol, 2-Methoxy-4-aminophenol, 3-Methoxy-4-aminophenol, 2,5-Dimethyl-4-aminophenol, 2-Methoxymethyl-4-aminophenol ausgewählt sind.

**7.** Zusammensetzung gemäß Anspruch 1 oder 2,
dadurch **gekennzeichnet**, daß
die Oxidationsfarbstoff-Vorstufen vom ortho-Typ aus o-Aminophenolen und o-Phenylendiaminen ausgewählt sind.

**8.** Zusammensetzung gemäß Anspruch 3,
dadurch **gekennzeichnet**, daß
die Doppelbasen Bisphenylalkylendiamine der Formel sind:

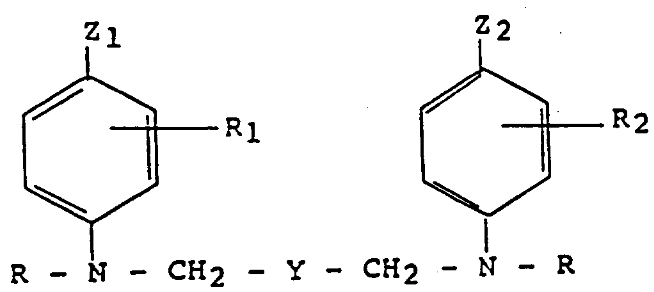

(IIbis)

worin:
$Z_1$ und $Z_2$, gleich oder verschieden, Hydroxy- oder $NHR_3$-Gruppen, worin $R_3$ ein Wasserstoffatom oder einen Niedrigalkylrest bedeutet,
$R_1$ und $R_2$, gleich oder verschieden, entweder Wasserstoffatome oder Halogenatome oder auch Alkylgruppen,
R ein Wasserstoffatom, eine Alkyl-, Hydroxyalkyl- oder Aminoalkylgruppe, deren Aminorest substituiert sein kann, und
Y einen Rest darstellen, ausgewählt aus der Gruppe, bestehend aus den folgenden Resten:
$-(CH_2)_n-$, $(CH_2)_n$, $-O-(CH_2)_{n'}-$, $-(CH_2)_{n'}-CHOH-$ $(CH_2)_{n'}-(CH_2)_n$,

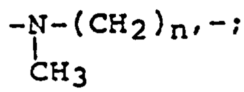

worin n eine ganze Zahl von 0 bis 8 und n' eine ganze Zahl von 0 bis 4 sind, wobei diese Base in Form ihrer Säureadditionssalze vorliegen kann.

**9.** Zusammensetzung gemäß Anspruch 8,
dadurch **gekennzeichnet**, daß
die Verbindungen der Formel (IIbis) aus N,N'-Bis(β-hydroxyethyl-N,N'-bis(4'-aminophenyl)-1,3-diamino-2-propanol, N,N'-Bis(β-hydroxyethyl)-N,N'-bis(4'-aminophenyl)ethylendiamin, N,N'-Bis(4-aminophenyl)-tetramethylendiamin, N,N'-Bis(β-hydroxyethyl)-N,N'-bis(4-aminophenyl)tetramethylendiamin, N,N'-Bis(4-methylaminophenyl)tetramethylendiamin, N,N'-Bis(ethyl)-N,N'-bis(4'-amino-3'-methylphenyl)-ethylendiamin ausgewählt sind.

**10.** Zusammensetzung gemäß jedem der Ansprüche 1 bis 9,
dadurch **gekennzeichnet**, daß
die Zusammensetzung auch, zusätzlich zu den heterozyklischen Kupplern der Formel (I), m-Diphenole, m-Aminophenole, m-Phenylendiamine, m-Acylaminophenole, m-Ureidophenole, m-Carbalkoxyaminophenole, α-Naphthol, 4-Hydroxyindol, β-Ketoverbindungen und Pyrazolone enthält.

**11.** Zusammensetzung gemäß jedem der Ansprüche 1 bis 10, dadurch **gekennzeichnet**, daß die Zusammensetzung auch Direktfarbstoffe enthält.

**12.** Zusammensetzung gemäß jedem der Ansprüche 1 bis 11, dadurch **gekennzeichnet**, daß die Oxidationsfarbstoffe vom para- und/oder ortho-Typ und die Kuppler in Mengenverhältnissen von 0,3 bis 7 Gew.%, bezogen auf Gesamtgewicht der Zusammensetzung, vorhanden sind.

**13.** Zusammensetzung gemäß jedem der Ansprüche 1 bis 12, dadurch **gekennzeichnet**, daß die Verbindung der Formel (I) in Mengenverhältnissen von 0,05 bis 3,5 Gew.%, bezogen auf Gesamtgewicht der Zusammensetzung, vorliegt.

**14.** Zusammensetzung gemäß jedem der Ansprüche 1 bis 13, dadurch **gekennzeichnet**, daß das zur Färbung der Fasern geeignete Milieu wässrig ist und einen pH von 8 bis 11 aufweist.

**15.** Zusammensetzung gemäß jedem der Ansprüche 1 bis 14, dadurch **gekennzeichnet**, daß die Zusammensetzung auch anionische, kationische, nichtionische, amphotere oberflächenaktive Mittel oder deren Mischungen enthält.

**16.** Zusammensetzung gemäß jedem der Ansprüche 1 bis 15, dadurch **gekennzeichnet**, daß die Zusammensetzung auch organische Lösungsmittel in Mengenverhältnissen von 1 bis 40 Gew.%, bezogen auf Gesamtgewicht der Zusammensetzung, enthält.

**17.** Zusammensetzung gemäß jedem der Ansprüche 1 bis 16, dadurch **gekennzeichnet**, daß die Zusammensetzung auch Verdickungsmittel, Antioxidantien, Eindringmittel, Sequestriermittel, Puffer und/oder Parfüm-Produkte enthält.

**18.** Zusammensetzung gemäß jedem der Ansprüche 1 bis 17, dadurch **gekennzeichnet**, daß sie in Form von Flüssigkeiten, Creme-Produkten, Gelen vorliegt und gegebenenfalls als Aerosole in Gegenwart eines Treibmittels zubereitet ist.

**19.** Verfahren zur Färbung keratinischer Fasern, insbesondere menschlicher Haare, dadurch **gekennzeichnet**, daß man eine in jedem der Ansprüche 1 bis 18 definierte Zusammensetzung auf die genannten Fasern, in Gegenwart einer oxidierenden Lösung, aufbringt, die Zusammensetzung 10 bis 40 Minuten lang in Kontakt mit den Fasern beläßt und im Anschluß an die Aufbringung eine Spülung der Haare, einen Waschvorgang, eine Spülung und einen Trockenvorgang folgen läßt.

**20.** Verfahren zur Färbung keratinischer Fasern, insbesondere menschlicher Haare, dadurch **gekennzeichnet**, daß man zu einem ersten Zeitpunkt die Oxidationsfarbstoff-Vorstufe vom para- und/oder ortho-Typ und, zu einem zweiten Zeitpunkt, mindestens einen heterozyklischen Kuppler der Formel (I) aufträgt, wobei die oxidierende Lösung entweder kurz vor dem Gebrauch in die zum zweiten Zeitpunkt aufgetragene Zusammensetzung eingebracht oder auch auf die Fasern zu einem dritten Zeitpunkt aufgetragen wird.

**21.** Verwendung einer Verbindung der Formel:

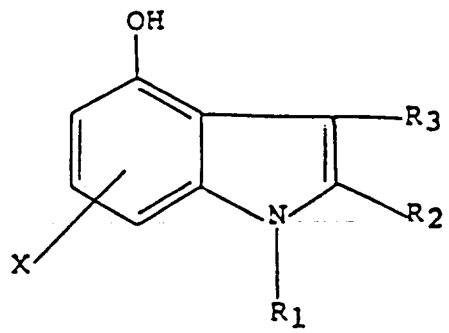

(I)

worin $R_1$ ein Wasserstoffatom, einen $C_{1-4}$-Alkylrest, $R_2$ ein Wasserstoffatom, einen $C_{1-4}$-Niedrigalkyl-, einen Carboxyl- oder Alkoxycarbonylrest, $R_3$ ein Wasserstoff- oder Halogenatom, einen $C_{1-4}$-Niedrigalkylrest, einen Carboxyl-, Alkoxycarbonyl- oder Formylrest und X ein Wasserstoffatom, einen $C_{1-4}$-Alkylrest, ein Halogenatom, einen $C_{1-4}$-Alkoxy-, einen Acetylamino- oder $C_{1-4}$-Dialkylaminomethylrest darstellen, wobei mindestens eine der Gruppen X, $R_1$, $R_2$, $R_3$ von Wasserstoff verschieden ist, sowie von deren Salzen als Kuppler für die Oxidationsfärbung keratinischer Fasern, wobei man mindestens eine Oxidationsfarbstoff-Vorstufe vom para- und/oder ortho-Typ zur Anwendung bringt.

**22.** Neue Verbindung, ausgewählt aus 4-Hydroxy-1,5-dimethylindol und 4-Hydroxy-2-carboxy-5-methylindol.